# EUROPEAN PATENT APPLICATION

(11) **EP 2 962 644 A1**
(43) Date of publication of application: **06.01.2016**
(21) Application number: 14175055.4
(22) Date of filing: 30.06.2014
(51) Int. Cl.: A61B 10/06, A61B 17/29

(54) **Endoscopic biopsy instrument**

(71) Applicant: BioScopeX ApS, 7600 Struer (DK)
(72) Inventor: Gundberg, Tomas, 4130 Viby Sjælland (DK); Kjeldsen, Ole, 7600 Struer (DK); Harboe, Henrik, 2840 Holte (DK)
(74) Representative: Guardian IP Consulting I/S

(57) **Abstract**

An endoscopic biopsy instrument 100 comprises an elongate flexible shaft 2, a biopsy device 1 arranged at a distal end of the shaft 2, and operating controls ar-ranged at a proximal end of the shaft 2. The endoscopic biopsy device 1 comprises at least one moveable element 3 that is operable for securing a tissue sample by means of one or more power actuators 6, 7 arranged in a distal portion of the endo-scopic instrument 100, wherein the power actuators 6, 7 comprise a shape memory alloy (SMA). The moveable element 3 is actuated by a set of counteracting SMA actuator wires 6/7 coupled to the moveable element 3 and arranged such that contraction of a drive SMA actuator wire 6 actuates a drive movement in a direction from a first position to a second position of the moveable element 3 and elongates a recovery SMA actuator wire 7, and accordingly contraction of the recovery SMA actuator wire 7 actuates a recovery movement in a direction from the second position to the first position of the moveable element 3 and elongates the drive SMA actuator wire 6.

## Description

The present invention relates to an endoscopic biopsy instrument comprising an elongate flexible shaft, a biopsy device arranged at a distal end of the shaft, and operating controls arranged at a proximal end of the shaft, wherein the endoscopic biopsy device comprises at least one moveable element that is operable for securing a tissue sample.

### BACKGROUND OF THE INVENTION

When performing endoscopy, it is often desired to take tissue samples at the same time. This may be achieved by advancing an endoscopic biopsy instrument through the instrument-channel of an endoscope to take a tissue sample. In order to be able to do so, the endoscopic biopsy instrument has to fulfil very stringent conditions as to the dimensions and remote operability through a long and narrow instrument-channel. The instrument-channel is typically between 2-4 mm, or even between 1-2 mm in ultrathin endoscopes. At the same time, endoscopes can have a length of about 1-2 m or even more. Furthermore when operated, the endoscope, and thus also the endoscopic biopsy instrument when inserted through the instrument channel, has often to follow a long tortuous path in order to reach the site of investigation and sample-taking. An endoscopic biopsy instrument therefore has a biopsy device for taking tissue samples, which is located at a distal end of an elongate, flexible shaft. The biopsy device and a distal portion of the elongate flexible shaft are dimensioned and configured to be inserted into and follow the instrument channel. The biopsy device and the distal portion of the shaft form a distal portion of the endoscopic biopsy instrument, which is adapted to fit in the instrument channel. The biopsy device is remotely operable by means of operating controls located at a proximal end of the elongate, flexible shaft extending outside the endoscope (and thus outside the patient), wherein the operating controls communicate with the biopsy device through the elongate, flexible shaft. Typically, the operating controls are combined in an operating handle at the proximal end of the endoscopic biopsy instrument for taking tissue samples from a bodily cavity, e.g. for taking tissue samples from the inside of the gastro-intestinal (GI) tract of a patient.

One problem of such endoscopic biopsy instruments that are intended for use in narrow instrument channels is to obtain a reliable remote operation, in particular when the endoscope is inserted into a body cavity along a tortuous path, such as when performing endoscopic biopsy in the GI-tract. Bending of the shaft along a tortuous path may, for example, cause problems of reliable mechanical power transmission. Such problems of reliable mechanical power transmission may include increased friction, inadvertent tensioning of a traction wire, offset/shifts in the relative positions of the distal end of traction wires, and may result in imprecise control of the sample taking procedure. Furthermore, the tough spatial constraints make it very difficult to implement more advanced/complex functionality that may require a plurality of wires and/or flexible push rods for the transmission of operation control forces to the biopsy device.

Object of the present invention is to provide an endoscopic biopsy instrument for securing and collecting tissue samples that overcomes problems of the prior art, provides an improvement or at least provides an alternative.

### SUMMARY OF THE INVENTION

According to one aspect, the invention relates to an endoscopic biopsy instrument comprising an elongate flexible shaft, a biopsy device arranged at a distal end of the shaft, and operating controls arranged at a proximal end of the shaft, wherein the endoscopic biopsy device comprises at least one moveable element that is operable for securing a tissue sample by means of one or more power actuators arranged in a distal portion of the endoscopic instrument, wherein the power actuators comprise a shape memory alloy (SMA), and wherein the moveable element is actuated by a set of counteracting SMA actuator wires coupled to the moveable element and arranged such that contraction of a drive SMA actuator wire actuates a drive movement in a direction from a first position to a second position of the moveable element and elongates a recovery SMA actuator wire, and accordingly contraction of the recovery SMA actuator wire actuates a recovery movement in a direction from the second position to the first position of the moveable element and elongates the drive SMA actuator wire.

The endoscopic biopsy instrument is for collecting tissue samples. In the context of the present application, the term "sample" refers to "tissue sample". The biopsy device, and accordingly the endoscopic biopsy instrument comprising the biopsy device, has a longitudinal axis defining an axial direction. When inserted in an endoscope, the longitudinal axis of the endoscopic device/instrument is parallel to the longitudinal axis of the instrument channel of the endoscope. Since the endoscope, when in use, has to follow a bent path, the term "parallel" is here to be understood when considering a particular point/section, i.e. the longitudinal axis of the endoscopic device/instrument essentially follows the longitudinal axis of the endoscope. Beyond the distal end interface of the endoscope or the instrument channel, the respective longitudinal axis may be extrapolated by the tangent to that longitudinal axis at the point of the end interface. The terms "proximal" and "distal" are defined with respect to an operator's view of the endoscopic instrument, i.e. "proximal" refers to the operating/controlling end, whereas "distal" refers to the functional end for insertion through the endoscope. Axial directions are parallel to the longitudinal axis, wherein a distal direction is the axial direction towards the distal end, and a proximal direction is the axial direction towards the proximal end. The terms "radial", "lateral" and "transverse" are also to be considered at a given point/section and refer to directions having a component perpendicular to the axial direction of that given point/section.

A distal portion of the endoscopic biopsy instrument comprises the biopsy device and at least a portion of the shaft. The distal portion of the endoscopic biopsy instrument is configured for insertion into the instrument channel of an endoscope and the operating controls are configured to communicate with the endoscopic biopsy device through the shaft to control operation of the endoscopic biopsy device. The endoscopic biopsy device comprises one or more moveable elements, such as jaws, that are actuated by means of one or more power actuators. "Moveable" refers to movement of said element(s) with respect to a body of the endoscopic biopsy device. By using power actuators to remotely control the moveable parts of the endoscopic biopsy device, such as for operating the jaws, the sample taking procedure can be programmed, automated, or at least semi-automated to perform an assisted procedure upon command- and/or trigger signals in response to user input. Thereby, sample taking may be simplified. For example, the opening and closing of the jaws could be operated by the operator from a foot-pedal thus leaving the hands of the operator free, e.g. to steer the endoscope itself. Preferably, the power actuators are electrically driven. Such electrical power actuators have the advantage that the power drive can be controlled directly using electronic signals provided by an electronic control circuit.

The power actuators are arranged in the distal portion of the endoscopic instrument, preferably in the endoscopic biopsy device itself. This arrangement reduces backlash in the remote operation of the moveable parts and solves issues of reliable mechanical power transmission due to bending of the instrument along a tortuous path, such as when performing endoscopic biopsy in the GI-tract. On the other hand, placing the entire power actuator mechanism in the distal portion of the endoscopic biopsy instrument subjects the power actuator mechanism to the above-mentioned stringent spatial constraints.

According to the preferred embodiment, this issue is addressed by using actuators comprising a shape memory alloy (SMA) as the motor component. The SMA is responsive to heat, wherein a shape, which has been trained into an element made of the SMA, is recovered by heating the SMA above a transition temperature where the SMA assumes a so-called high-temperature phase, in which the shape memory is activated. Upon cooling, the SMA assumes a low-temperature phase without shape memory, the material "relaxes" and the SMA element may easily be deformed. Preferably, the SMA is electrically conductive with an electrical resistance such that the shape memory of an SMA element can be switched on by passing a heating current through the SMA element to bring it into the high-temperature phase, and switched off by switching off the heating current and cooling the SMA material to bring it into the low-temperature phase. The transition between the high-temperature phase and the low-temperature phase occurs over a transition regime exhibiting hysteresis, wherein activation of the shape memory by heating requires higher temperatures than the temperatures at which relaxation of the material occurs during the cooling phase.

According to the preferred embodiment, the one or more power actuators are actuator wires made of an SMA responsive to heat, wherein the actuator wires contract upon heating the SMA above a transition temperature, where the SMA is in a high temperature phase, and slacken upon cooling the SMA below a transition temperature, where the SMA is in a low temperature phase. In order to achieve a contraction next time the shape memory is activated, the slackened actuator wire has to be elongated in the low temperature phase by applying a stretching force. The hysteresis effect of the SMA actuator wires shows e.g. in plots of temperature versus strain in the SMA actuator wire, wherein contraction upon heating from the low temperature phase to the high temperature phase (heating branch) is achieved above an upper transition temperature, and wherein elongation upon cooling from the high temperature phase to the low temperature phase (cooling branch) under tensile load is achieved below a lower transition temperature. Both in the heating branch and in the cooling branch, the actuator wire exhibits in a transition regime around the respective transition temperature a sharp but monotonous strain response, where the strain strongly increases with increasing temperature.

The operation temperature range of a given SMA depends on the particular composition and working of the SMA material. The temperature behaviour of a given, commercially available actuator is typically available in a corresponding technical data sheet, for example in the form of temperature vs. strain characteristics or tabulated values regarding the transition temperature range and further technical data. Typical operation temperature values characterizing a given SMA material, around which the material undergoes phase transition, are about 70 degrees C, or about 90 degrees C, but other material compositions with different operating temperatures may easily be selected by the skilled person and are commercially available. When selecting a material for the SMA actuators, it should be taken into account that the biopsy device of the endoscopic instrument is usually intended for operation in a thermal environment with a predetermined temperature range as defined by the body temperature of the patient. This influences the heat transfer to and from the SMA actuators and consequently both the heating power required for raising the temperature of the SMA actuators to undergo transition, as well as the time for cooling the SMA actuators down again. In order to be able to control the temperature of the SMA actuators by heating (rather than cooling), the transition regime of the SMA actuator should be chosen well above the temperature of the environment in which it is to be operated. At the same time, the need for dissipating heat to that environment also requires attention to potential thermal damage to adjacent tissue that may be caused by the operation of the SMA actuator wires in close vicinity. SMA actuators operating at lower temperatures closer to the temperature of the environment of intended use carry a lower risk of thermal damage to surrounding tissue at the expense of longer cooling times. On the other hand, SMA actuators operating at higher temperatures, further away from the temperature of the environment of intended use, have the advantage that the larger difference between the SMA actuator and its surroundings brings about a more efficient and thus more rapid cooling. This, again, is at the expense of a higher risk for thermal damage to the surrounding tissue, and requires more electrical energy to be dissipated by the system and thus also results in higher energy consumption.

To avoid that surrounding tissue is thermally affected/burned by SMA actuator wires, different measures may be taken. For example, one measure may be providing insulation and/or heat shielding, e.g. by arranging the SMA actuators inside a tubular outer screen. Thereby any direct heat transfer from the SMA actuators to the tissue by heat conduction and/or radiation is avoided or at least reduced. This might have the disadvantage that the cooling of the SMA actuators becomes less efficient. However, thereby the immediate thermal environment of the SMA actuators is better defined also in different operational conditions/situations, thus allowing for a more predictable temperature control. This improves precision and reliability when operating the endoscopic instrument. Alternatively or supplementary thereto a further measure may be reducing the resistance of any portions of the current conducting linkage between the moveable elements and the body of the endoscopic instrument, which are exposed to tissue during operation. Thereby the Joule heating of the exposed portions of the SMA actuator mechanism is reduced, to a degree, where thermal exposure is no longer harmful (e.g. by thickening a SMA actuator wire in these regions , cramping together with a different material, extending the levers/linkage so as to keep the SMA actuators inside the housing).

Advantageously, the one or more SMA actuator wires are straight wires extending parallel along the axial direction. Thereby a lateral space saving configuration is achieved. Preferably in this parallel arrangement, the SMA actuator wires are arranged peripherally as seen in a transverse cross-section. Thereby a lateral space saving configuration is achieved keeping a central clearance, e.g. for the passage of auxiliary devices or advantageously for arranging a reservoir for the safe-keeping of multiple samples collected by means of the biopsy device.

As mentioned above, the biopsy device has moveable elements that are actuated by the power actuators. Examples for such moveable elements are jaws, collecting members and/or retaining elements, which are used for securing and collecting tissue samples from a site of interest. The movement of the moveable elements may be defined as a stroke between a first position and a second position. The stroke of the moveable element has a drive portion and a recovery portion, wherein the term "drive" denotes movements in a direction from the first position to the second position, and the term "recovery" denotes movements in a direction from the second position to the first position, i.e. opposite to the drive direction. In the case of jaws, the first position may be an OPEN position, such as fully open or at least partially open, where cooperating jaws are spaced apart from each other, and the second position may be a CLOSED position where the cooperating jaws are brought together. In this case, the drive movement is closing the jaws by moving the jaws from the OPEN position to the CLOSED position, and the recovery movement is opening the jaws by moving the jaws from the CLOSED position to the OPEN position.

The drive movement of the moveable element is actuated by the temperature-dependent contraction of a first SMA actuator wire when this is in the high temperature phase (activated state). The first SMA actuator thus exerts a drive force on the moveable element. The recovery movement of the moveable element is actuated by a recovery element exerting a recovery force on the moveable element in a direction opposite to the drive force. The recovery force thus induces elongation of the first SMA actuator wire when this is in the low temperature phase (relaxed state). Such a recovery element may be a passive bias element that exerts a bias force on the moveable element in the recovery direction. Advantageously according to one aspect of the present invention, the recovery element is a second SMA actuator wire that exerts a recovery force resulting from the temperature-dependent contraction of the second SMA actuator wire when this is in the high temperature phase (activated state). The second SMA actuator thereby counteracts the first SMA actuator. To achieve proper drive and recovery movements, the first and second SMA actuators therefore have to be controlled so as to cooperate such that when one is contracted, the other one is slackened.

At least one moveable element is actuated by counteracting SMA actuator wires coupled to the moveable element. The counteracting SMA actuator wires are arranged such that contraction of a drive SMA actuator wire in the high temperature phase actuates a drive movement in a direction from a first position to a second position of the moveable element and elongates a recovery SMA actuator wire in the low temperature phase, and accordingly contraction of the recovery SMA actuator wire in the high temperature phase actuates a recovery movement in a direction from the second position to the first position of the moveable element and elongates the drive SMA actuator wire in the low temperature phase. Consequently the actuation forces for both the drive movement and the recovery movement are controlled directly by the respective drive and recovery SMA actuator wires. This allows, for example, for reducing the counterforce during the drive phase, since a spring loaded recovery bias can be reduced or even omitted. Using SMA actuator wires allows for a lateral space saving design for the actuator mechanism so as to reduce the foot print taken up by the actuator mechanism as seen in transverse cross-sections of the biopsy device. For example, the SMA actuator wires may be arranged in the distal end portion of the biopsy instrument essentially parallel to the longitudinal direction thereof. Advantageously, the SMA actuator wires may be arranged peripherally as seen in a transverse cross-section, so as to leave a central lumen for the passage of other components, devices, or for storage space for collecting multiple samples.

In the following, further advantageous embodiments are described. Amongst others, these advantageous embodiments address issues to be observed when using SMA actuator wires in order to avoid deteriorated performance and fatal failure of the actuator wire, such as excessive heating, excessive strain exerted on the actuator wire in the high-temperature phase, overstretching of the actuator wire in a transition regime, and excessive elongation in the low-temperature phase. In particular, in the design with counteracting actuator wires a proper control of the strain and the stress in the SMA actuator wires is advantageous in order to avoid failure of the device by overstretching or even breakage of SMA actuator wires. The strain in the SMA actuators may e.g. be controlled by precise control of the temperature of the SMA actuators depending on their actual elongation throughout a cycle of actuation. Measures for improving the lifetime of the SMA actuators are further detailed in the following. They concern temperature control during operation, mechanical strain relief and/or load limitation, and combinations thereof.

Advantageously according to one embodiment of the endoscopic biopsy instrument, the operation of the moveable elements is current controlled. The current control is achieved by selectively controlling a heating current passed through the SMA actuator wires to control their temperature, wherein the SMA is operated in the transition regime so as to induce a current controlled strain in the SMA/actuator wire. The current control has the advantage that is facilitates a remote, automated and/or an electronically controlled operation.

Further according to one embodiment of the endoscopic biopsy instrument, the drive and recovery SMA actuator wires are anchored to the body of the endoscopic biopsy instrument via one or more elastic elements. To achieve an actuation of a drive or recovery movement of the moveable element by contraction of the respective SMA actuator wire, one end of the SMA actuator wire has to be connected to the moveable element, such as to a lever of a jaw, and the other end has to be anchored to the body of the biopsy instrument. The one or more elastic elements provide a "floating" anchoring of the SMA actuator wires to the body of the endoscopic biopsy instrument, wherein the coupling of the floating anchor to the body is determined by the elasticity of the one or more elastic elements. Preferably, the elasticity of the one or more elastic elements is adapted to provide strain relief limiting the load on the SMA actuator wires under contraction, such that excessive strain that otherwise might result in an overstretching or even breakage of the SMA actuator wire is absorbed by the deformation, e.g. elongation or compression, of the elastic element. By adding an elastic element to the link between the moveable element and the body of the endoscopic device, in series with the SMA actuator of that link, the counteracting SMA actuator mechanism becomes more tolerant as to the precision of the actual temperature control of the SMA-actuators.

Further according to one embodiment of the endoscopic biopsy instrument, the drive and recovery SMA actuator wires are coupled to the one or more elastic elements via a common yoke. The common yoke provides a common "floating" anchoring of the counteracting drive and recovery SMA actuator wires via the one or more elastic elements to the body of the endoscopic biopsy instrument. Thereby, a combined strain relief/load limitation mechanism is achieved, which is operational for both drive and recovery.

Further according to one embodiment of the endoscopic biopsy instrument, at least one elastic element is a spring, preferably an axially aligned helical spring. This allows for a compact design, which is compatible with the axially aligned, elongated geometry of the endoscopic device, keeping a central lumen clear. Depending on the particular device the helical spring may be a compression spring, a tension spring, or the elastic elements may comprise a combination of compression and tension springs.

Further according to one embodiment of the endoscopic biopsy instrument, the one or more elastic elements are arranged to apply a tensioning force on the SMA actuator wire. Thereby backlash in the mechanical system is reduced. Furthermore, the tensioning force contributes to stretching the SMA actuator wires during cooling to ensure appropriate elongation of the SMA actuator wires in the low temperature phase (relaxed state). Thereby a simple, compact and reliable configuration with reduced/low backlash is achieved. The floating anchor design is also robust against unpredictable events, such as mechanical disturbances blocking the movement of the moveable element, and tolerant as to the precision of the actual temperature control of the SMA-actuators. Furthermore the force exerted by the moveable element, such as the gripping force exerted by jaws on a tissue sample, can be limited to a desired maximum.

According to some embodiments, the endoscopic biopsy instrument may be configured for advanced functionality involving a more complex mechanical design with more moving parts as compared to a single sample biopsy forceps function. For example, the endoscopic biopsy device may be configured for collecting multiple tissue samples. In such an embodiment the moveable elements may comprise jaws, a collecting member, and/or retaining elements for collecting secured tissue samples. Advantageously, one or more of the moveable elements of an endoscopic biopsy device are actuated by means of SMA-actuator wires. Preferably, all moveable elements requiring actuation are actuated by means of SMA-actuator wires.

Further according to one embodiment of the endoscopic biopsy instrument, contraction and/or elongation of the SMA actuator wire is controlled by controlling a heating current passed through the SMA actuator wire. Thus contraction and/or elongation of the drive SMA actuator wire is controlled by controlling a heating current passed through the drive SMA actuator wire, and/or wherein contraction and/or elongation of the recovery SMA actuator wire is controlled by controlling a heating current passed through the recovery SMA actuator wire. By passing a current through the SMA actuator wire, the SMA actuator wire may be heated. The SMA actuator wire then contracts upon heating above the transition temperature and the actuator wire elongates when under tensile load upon cooling below a transition temperature, wherein the actuator wire exhibits a hysteresis effect as discussed above. Cooling of the SMA actuator wire is mainly by heat transfer to the environment. The heat dissipation can be counteracted by applying a heating current, e.g. to slow down the cooling rate, or to stabilize the temperature of the SMA actuator wire. Heating and cooling can thus be controlled by controlling the current passed through the SMA actuator wire. As mentioned above, embodiments with a strain relief anchoring of the counteracting SMA actuators, e.g. where the SMA actuator wires are anchored to the body of the endoscopic biopsy instrument via one or more elastic elements, are less sensitive to the precision of the temperature control than embodiments without any such strain compensation device.

Further according to one embodiment of the endoscopic biopsy instrument, the heating current is controlled in dependence of the temperature of the SMA actuator wire. Thereby a precise control of the strain and stress in the SMA actuator wires and thus a precise control of the actuation mechanism of the moveable elements in the endoscopic biopsy instrument may be achieved.

Further according to one embodiment of the endoscopic biopsy instrument, the temperature of the SMA actuator wire is determined by measuring the temperature-dependent resistance of the SMA actuator wire. The temperature dependent resistance of the SMA actuator wire may e.g. be determined directly from simultaneous measurements of current and voltage, which may be converted to a temperature value by look-up/interpolation of calibration data determined beforehand. By directly measuring the temperature, rather than estimating or deriving it indirectly, e.g. from current data and system parameters, a better precision of the actuation control is achieved.

Further according to one embodiment of the endoscopic biopsy instrument, the heating current comprises pulses, wherein the pulsed heating current is defined by a sequence of ON states where the heating current is switched on, separated by OFF states where the heating current is switched off. Preferably, the heating current is switched periodically on and off. Furthermore, the time scale of the switching, e.g. as characterized by the time between subsequent pulses, is shorter than the time scale of thermal stabilization in the SMA actuator wire, as e.g. characterized by a characteristic step response time of the SMA actuator wire temperature to a switching between ON and OFF states.

Further according to one embodiment of the endoscopic biopsy instrument, the heating current is controlled by pulse width modulation. The temperature of the actuator may be controlled by varying the pulse width of the ON state, the pulse width of the OFF state, and/or the duty cycle of subsequent ON and OFF states. Each pulse period comprises a full ON state cycle followed by a full OFF state cycle.

Further according to one embodiment of the endoscopic biopsy instrument, the temperature of the actuator wire is probed during OFF states in between ON states. The resistance measurement is performed using a small probe current, which allows probing the temperature also during cool-down.

Further according to one embodiment of the endoscopic biopsy instrument, the moveable elements comprise jaws. The jaws may be pivoted jaws. Using counteracting sets of drive and recovery SMA actuator wires, the jaws are operated to actuate both the closing movement (drive) and the opening movement (recovery). Further according to one embodiment of the endoscopic biopsy instrument, the operating controls comprise one or more of a foot pedal, a trigger button, a toggle switch, a joy stick, and a computer comprising a gesture based human-machine interface (HMI). Thereby, the operation of the endoscopic biopsy instrument is simplified and thus more user friendly.

Further according to one embodiment of the endoscopic biopsy instrument, the operating controls are detachably coupled to the proximal end of the shaft by means of a connector. The detachable coupling allows for an easy exchange of the distal portion of the endoscopic biopsy instrument. By choosing to locate the detachable coupling at the proximal end, the biopsy device and the shaft form a unit including the entire distal portion intended for insertion into the instrument channel of an endoscope, which can easily be detached and replaced by a new one. This allows e.g. for using different types of biopsy devices with the same operating controls during a given procedure or simply to increase sample collecting capacity in a procedure by using a plurality of the same type of biopsy device.

Further according to one embodiment of the endoscopic biopsy instrument, the connector comprises an electrical interface configured for transmitting electrical power and/or control signals to the power actuators. Integrating an electrical interface in the detachable connector between the proximal end of the shaft and the operating controls allows for an easy and rapid replacement of the biopsy device and shaft unit. The electrical interface is preferably standardized for a given family of biopsy devices to achieve modularity. The detachable connector may further comprise mechanical, optical and/or electronic identification means that allow the user and/or an electronic unit and/or a computer to detect/identify the biopsy device and shaft unit; identify e.g. compatibility between the biopsy device/shaft unit and the operating controls; or even prevent incompatible combinations.

### BRIEF DESCRIPTION OF THE DRAWINGS

Preferred embodiments of the invention will be described in more detail in connection with the appended drawings, which show in
- FIG. 1: a side cross-sectional detail of the endoscopic biopsy instrument showing the biopsy device and a distal end of the shaft,
- FIG. 2: a corresponding top cross-sectional detail of the endoscopic biopsy instrument of Fig.1,
- FIG. 3: a transverse cross-sectional view of the endoscopic instrument taken at the common yoke of the actuator anchoring mechanism seen in a distal direction as indicated by line III-III in Fig.1,
- FIG. 4: a side cross-sectional detail of the floating anchoring mechanism of the counteracting SMA actuator wires in the endoscopic biopsy instrument of Fig.1, and
- FIG. 5,: a further side cross-sectional detail of the endoscopic biopsy instrument showing the biopsy device and a distal end of the shaft.

### DETAILED DESCRIPTION

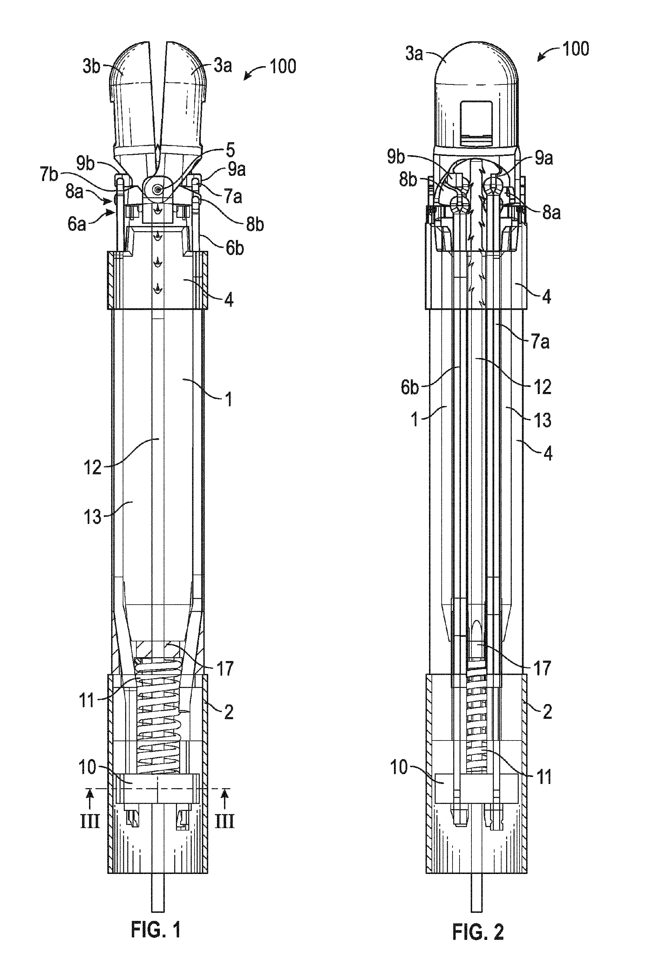

Figs.1-5 show different cross-sectional views of details of a distal end portion of one embodiment of an endoscopic biopsy instrument 100 where a biopsy device 1 is attached to the distal end of a shaft 2. The biopsy device 1 comprises an elongate tubular body 4 extending in an axial direction from a distal end to a proximal end, wherein the axial direction is parallel to the longitudinal axis of the tubular body 4. The tubular body 4 has an internal cavity with an opening at the distal end. The internal cavity defines a collecting chamber 13 for receiving multiple tissue samples.

At a distal end, the biopsy device 1 comprises two half-shell shaped jaws 3a, 3b with their concave side facing to each other. The jaws 3a, 3b are hinged to the distal end of the body 4 by means of a pivot joint 5 with a pivot axis perpendicular to the cross-sectional plane of Fig.1. The jaws 3a, 3b are further moveable with respect to each other between an OPEN position, and a CLOSED position. In the CLOSED position, the jaws 3a, 3b define a sampling chamber.

The jaws 3a, 3b may be remotely actuated to open and close by means of SMA actuator wires 6a/7a, 6b/7b. The jaws 3a, 3b are thus operable to grasp around a sample and secure the sample inside the sampling chamber.

The movement of the jaws 3a, 3b is actuated by means of respective sets of counteracting SMA actuator wires 6a/7a, 6b/7b extending from the distal jaws 3a, 3b, along the longitudinal direction of the endoscopic instrument 100, to a common yoke 10 of a proximally arranged floating anchoring mechanism, which is connected to a body portion 4 of the endoscopic biopsy instrument 100 via a compression spring 11. Each of the SMA actuators 6a/7a, 6b/7b are here formed by a SMA actuator wire laid double, wherein the wire is bent and crimped at the distal end to form a loop engaging an eyelet on a respective drive/recovery lever 8a/9a, 8b/9b of the jaws 3a, 3b, and wherein the two ends of each wire 6a/7a, 6b/7b are crimped together at the proximal end and engage a respective slot 14a/15a, 14b/15b on the common yoke 10. Forming the SMA actuator as a double wire, or alternatively with even more wires acting in parallel, has advantages of thermal management, since the dual/multiple wire strands have a faster thermal response to changing/switching of a heating current passed there through, in particular during cooling of the SMA actuator wire as compared to a single wire dimensioned for the same actuation force. A suitable SMA actuator wire is for example available from DYNALLOY under the brand name FLEXINOL, the technical details of which are described in the datasheet "Technical Characteristics of FLEXINOL® Actuator Wires" (Dynalloy datasheet F1140Rev H).

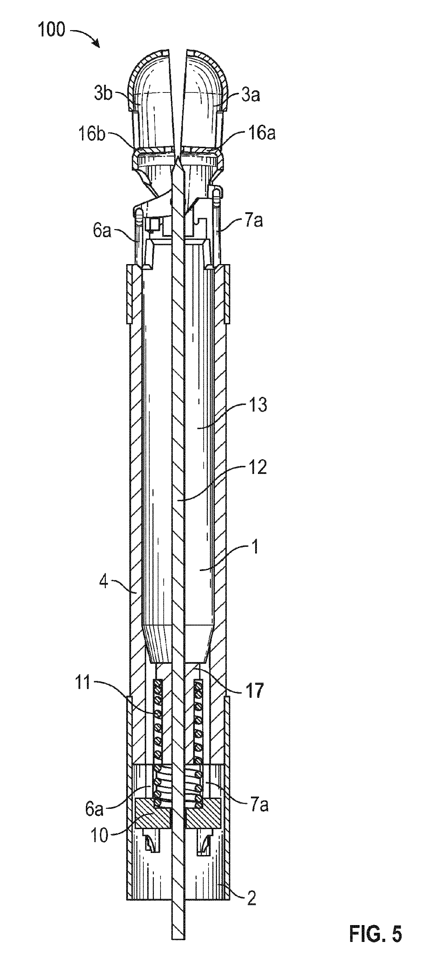

In Figs.1-5, the biopsy device is shown in an IDLE position between the OPEN and the CLOSED positions where both drive and recovery SMA actuator wires 6a/7a, 6b/7b are relaxed and extended in the low temperature phase, and where the compression spring is also in its relaxed state. In the IDLE position, the jaws are slightly open as best seen in Figs. 1 and 5.

A closing movement of the jaws 3a, 3b in a direction from the OPEN position towards the CLOSED position is actuated by passing a heating current through the drive SMA actuator wires 6a, 6b so as to induce a contraction, thereby exerting a pulling force in a proximal direction on the drive levers 8a, 8b of the respective jaws 3a, 3b. The pulling force is transferred to the recovery SMA actuator wires 7a, 7b via the common yoke 10 of the floating anchor mechanism, and further to the recovery levers 9a, 9b of the respective jaws 3a, 3b to which the distal end of the recovery SMA actuator wires is attached. The contraction of the drive actuator wires 6a, 6b thus works against and stretches the recovery SMA actuator wires 7a, 7b, wherein the floating anchoring mechanism acts as a tensioner. Accordingly, an opening movement of the jaws 3a, 3b in a direction opposite to the closing movement is actuated by applying a heating current of the recovery SMA actuator wires 7a, 7b so as to induce a contraction of the recovery SMA actuator wires to apply an proximally directed pulling force on the recovery levers 9a, 9b that exceeds the proximally directed pulling force acting on the drive levers 8a, 8b. Due to the coupling of the drive and recovery via the floating anchoring mechanism, the relative elongation/contraction of the drive SMA actuator wires with respect to the counteracting recovery SMA actuator wires decides whether the jaws 3a, 3b open or close. The relative elongation or contraction is controlled by controlling the temperatures of the drive and recovery SMA actuator wires.

As mentioned above, the common yoke 10 is attached to a body portion of the endoscopic biopsy instrument 100 via the compression spring 11, which is adapted for taking up axial forces by pressing against a seat 17 at the proximal end of the biopsy device body 4. A shortening of the total length of the counteracting SMA actuator wires 6a/7a, 6b/7b, i.e. a reduction of the sum of the drive and recovery SMA actuator wire lengths is compensated for by an axial displacement of the common yoke 10 in a distal direction against the spring force of the compression spring 11. The stretching forces acting on the drive and recovery SMA actuator wires 6a/7a, 6b/7b are thus limited by the spring constant of the compression spring 11. Consequently, also the opening and closing forces acting upon the jaws 3a, 3b are limited by means of the compression spring 11.

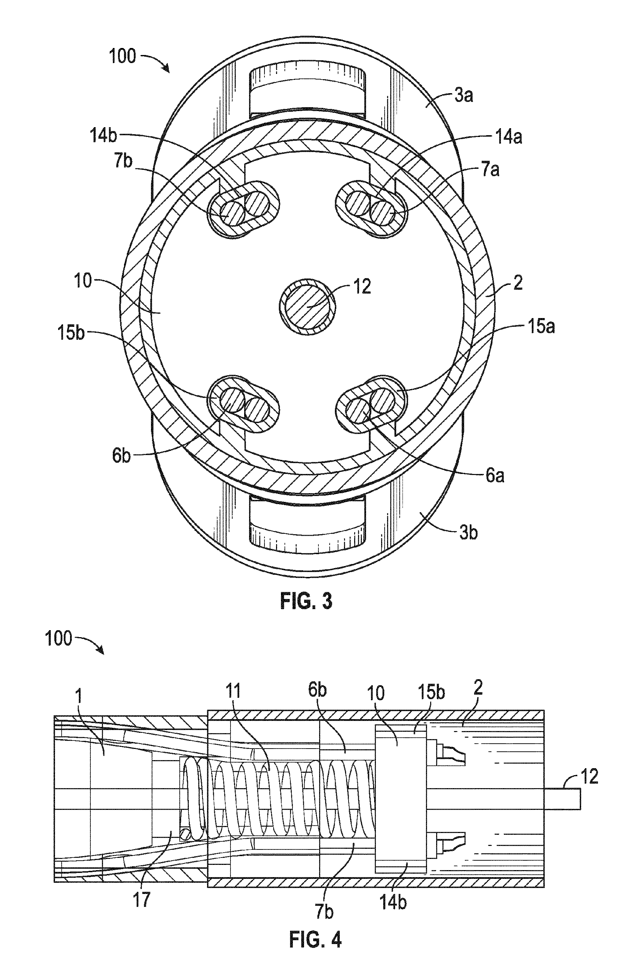

In the particular embodiment shown here, the endoscopic biopsy instrument with biopsy device 1 further comprises a needle-shaped collecting member 12. The collecting member 12 is arranged parallel to the axial direction, on the central axis of the tubular body 4. At a distal end, the collecting member 12 has a needle tip pointing in the distal direction. Preferably, the collecting member is provided with barbs/retro-serrations/protuberances, as shown in the drawing. The tips of the barbs and/or retro-serrations are designed to point in a proximal direction away from the needle tip, so as to prevent transfixed samples from sliding off the needle. The collecting member 12 is slidable in the axial direction. The collecting member 12 may thus be operated in a linear translational movement along the axial direction. The collecting member 12 is deployed by moving it in the distal direction, and retracted by moving it in the proximal direction. On a proximal portion of the jaws 3a, 3b, retaining elements 16a, 16b are provided pointing radially inward (see Fig.5). The retaining elements 16a, 16b engage when the jaws 3a, 3b are in the CLOSED position, and disengage when the jaws 3a, 3b are in the OPEN position. Advantageously, the retaining elements 16a, 16b may be produced from the peripheral wall of the half-shell shaped jaws 3a, 3b, e.g. by cutting an appropriate shape, leaving a transversely oriented side of the shape connected, and bending the shape towards the inside of the half-shell to point radially inward. When engaged, the retaining elements 3a, 3b define a proximal end of the sampling chamber and form a transversely oriented partition separating the sampling chamber from the collecting chamber 13. The retaining elements 16a, 16b are shaped, when engaged, to leave a central aperture for the collecting member 12 to pass. When disengaged, the retaining elements 16a, 16b open a passage for tissue samples to enter the collecting chamber 13 for storage. The endoscopic biopsy instrument 100 is thus adapted for collecting multiple tissue samples. To give place for the system for the collection of multiple tissue samples, the SMA actuator wires 6a/7a, 6b/7b are arranged peripherally to extend axially alongside the sampling chamber 13, the floating anchoring mechanism with common yoke 10 and compression spring 11 is arranged at the proximal end of the sampling chamber 13, and the common yoke 10 has a central aperture allowing for the collecting member 12 to pass (see e.g. Figs. 3 and 5).

### List of Reference Numbers

- 100: endoscopic biopsy instrument
- 1: biopsy device
- 2: shaft
- 3, 3a, 3b: moveable element, jaws
- 4: body
- 5: hinge
- 6, 6a, 6b: drive SMA actuator wire
- 7, 7a, 7b: recovery SMA actuator wire
- 8a, 8b: drive lever
- 9a, 9b: recovery lever
- 10: common yoke
- 11: compression spring
- 12: collecting member
- 13: collecting chamber
- 14a, 14b: slot
- 15a, 15b: slot
- 16a, 16b: retaining element
- 17: seat

## Claims

1. Endoscopic biopsy instrument (100) comprising an elongate flexible shaft (2), a biopsy device (1) arranged at a distal end of the shaft (2), and operating controls arranged at a proximal end of the shaft (2), wherein the endoscopic biopsy device (1) comprises at least one moveable element (3) that is operable for securing a tissue sample by means of one or more power actuators (6, 7) arranged in a distal portion of the endoscopic instrument (100), wherein the power actuators (6, 7) comprise a shape memory alloy (SMA), and wherein the moveable element (3) is actuated by a set of counteracting SMA actuator wires (6/7) coupled to the moveable element (3) and arranged such that contraction of a drive SMA actuator wire (6) actuates a drive movement in a direction from a first position to a second position of the moveable element (3) and elongates a recovery SMA actuator wire (7), and accordingly contraction of the recovery SMA actuator wire (7) actuates a recovery movement in a direction from the second position to the first position of the moveable element and elongates the drive SMA actuator wire (6).

2. Endoscopic biopsy instrument according to claim 1, wherein the drive and recovery SMA actuator wires (6/7) are anchored to the body (4) of the endoscopic biopsy instrument (1) via one or more elastic elements (11).

3. Endoscopic biopsy instrument according to claim 2, wherein the drive and recovery SMA actuator wires (6/7) are coupled to the one or more elastic elements (11) via a common yoke (10).

4. Endoscopic biopsy instrument according to any one of claims 2 or 3, wherein at least one elastic element is a spring (11), preferably an axially aligned helical spring.

5. Endoscopic biopsy instrument according to any one of claims 2 - 4, wherein the one or more elastic elements are arranged to apply a tensioning force on the SMA actuator wire (6, 7).

6. Endoscopic biopsy instrument according to any of the preceding claims, wherein contraction and/or elongation of the drive SMA actuator wire (6) is controlled by controlling a heating current passed through the drive SMA actuator wire (6), and/or wherein contraction and/or elongation of the recovery SMA actuator wire (7) is controlled by controlling a heating current passed through the recovery SMA actuator wire (7).

7. Endoscopic biopsy instrument according to claim 6, wherein the heating current is controlled in dependence of the temperature of the SMA actuator wire (6, 7).

8. Endoscopic biopsy instrument according to claim 7, wherein the temperature of the SMA actuator wire (6, 7) is determined by measuring the temperature-dependent resistance of the SMA actuator wire (6, 7).

9. Endoscopic biopsy instrument according to any one of claims 6 - 8, wherein the heating current comprises pulses.

10. Endoscopic biopsy instrument according to claim 9, wherein the heating current is controlled by pulse width modulation.

11. Endoscopic biopsy instrument according to any of the preceding claims, wherein the moveable elements (3) comprise jaws (3a, 3b).

12. Endoscopic biopsy instrument according to any of the preceding claims, wherein the operating controls comprise one or more of a foot pedal, a trigger button, a toggle switch, a joy stick, and a computer comprising a gesture based human-machine interface (HMI).

13. Endoscopic biopsy instrument according to any of the preceding claims, wherein the operating controls are detachably coupled to the proximal end of the shaft (2) by means of a connector.

14. Endoscopic biopsy instrument according to claim 13, wherein the connector comprises an electrical interface configured for transmitting electrical power and/or control signals to the power actuators (6, 7).
